# EUROPEAN PATENT APPLICATION

(11) **EP 4 744 588 A1**
(43) Date of publication of application: **20.05.2026**
(21) Application number: 25209934.6
(22) Date of filing: 21.10.2025
(51) Int. Cl.: A61B 5/145

(54) **APPLICATOR FOR INSERTING SENSOR UNIT**

(30) Priority: 18.11.2024 KR 20240164545
(71) Applicant: I-SENS, INC., Seoul 06646 (KR)
(72) Inventor: CHOI, Hyun Ho, 06646 Seoul (KR)
(74) Representative: BCKIP Part mbB

(57) **Abstract**

Provided in the present disclosure is an applicator, including a case which forms an inner space, includes an opening part opened to one side, and includes a button hole formed by penetrating at least a portion of an outer circumferential surface, an operation part which seals the button hole, and a plunger which is disposed in the inner space of the case, and is configured to move a sensor unit towards the opening part by an operation of the operation part, wherein the operation part includes a button part of which at least a partial area is disposed in the inner space through the button hole, and an edge groove part which is configured to facilitate movement of the button part.

## Description

### CROSS-REFERENCE TO RELATED APPLICATION(S)

This application claims the benefit of Korean Patent Application No. 10-2024-0164545, filed on November 18, 2024, in the Korean Intellectual Property Office.

### FIELD OF THE INVENTION

Example embodiments relate to an applicator.

### BACKGROUND

Modem people suffer from various illnesses, and, in order to appropriately treat or manage the various illnesses, a means for obtaining biometrics of a human is required.

A representative example is diabetes. In order to appropriately manage diabetes, a means for obtaining blood sugar information, which is biometrics of a diabetic, is required.

Recently, a continuous glucose monitoring (CGM) device, which is inserted into a human body and measures a blood sugar level by an interval of few minutes, is being developed. The CGM device may continuously measure blood sugar after a sensor in a needle shape is inserted into a relatively less painful part such as an abdomen and an arm, in order to minimize pain and repulsion of a user caused by blood sampling.

The CGM device is configured by including a sensor unit which is attached to a body of a user to measure blood sugar information of the user before transmitting the measured blood sugar information, and a terminal which provides the blood sugar information received from the sensor unit to the user in order for the user to monitor or manage the blood sugar information of the user.

Here, the sensor unit is constituted with a sensor module which is attached by being inserted into a skin of the body of the user to measure blood sugar from a bodily fluid of the user, and a transmitter which transmits a blood sugar level which is measured by the sensor module to the terminal, wherein a probe formed in a needle shape to be inserted into subcutaneous fat to extract an intracellular fluid is provided at the sensor module.

An applicator is used for inserting and attaching the sensor unit in and to a human body.

The applicator is configured to insert and attach the sensor unit in and to the human body, and thus, inserts and attaches the sensor unit in and to the human body by an operation of the user, and, as the applicator is separated and removed from the human body in a state in which the sensor unit is inserted and attached in and to the human body, only the sensor unit remains in a state of being attached to the human body.

Because a portion of the sensor unit is inserted into the human body, the sensor unit installed in the applicator and an inner space of the applicator should be maintained in a sterilized state starting at an operation of manufacture until a time before being used by a consumer, and should be maintained in a dehumidified state to preserve electronic components. At the same time, the inner space of the applicator may be maintained in a sealed state from the outside to maintain the sterilized and dehumidified state.

In order to maintain the sterilized, dehumidified and sealed state, various packaging technologies are being applied to the applicator, and simplifying a packaging and reducing a size are becoming one of the goals of a manufacturer of the applicator.

Meanwhile, a user may operate (trigger) the applicator by pressing a button. Because a button part is disposed to operate a trigger in a case by being located on the outside of the case of the applicator, a hole, through which the button enters and exits, should be formed at a portion of the case. Nevertheless, a problem arises in that the corresponding hole should be sealed, because the inner space of the applicator should be sealed.

### SUMMARY OF THE INVENTION

An aspect provides an applicator which may maintain an inner space in a sterilized, dehumidified, and sealed state.

Another aspect also provides an applicator which may simplify a packaging which may maintain an inner space of an applicator in a sterilized, dehumidified, and sealed state, and reduce a size of a packaging.

According to an aspect, there is provided an applicator, including a case which forms an inner space, includes an opening part opened to one side, and includes a button hole formed by penetrating at least a portion of an outer circumferential surface, an operation part which seals the button hole, and a plunger which is disposed in the inner space of the case, and is configured to move a sensor unit towards the opening part by an operation of the operation part, wherein the operation part includes, a button part of which at least a partial area is disposed in the inner space through the button hole, and an edge groove part which is configured to facilitate movement of the button part.

According to another aspect, the edge groove part is formed to be recessed towards the inner space from the outer circumferential surface.

According to another aspect, at least a portion of the edge groove part is formed along a periphery of the button part.

According to another aspect, the edge groove part includes a first extension part which extends towards the inner space from the button part, and a second extension part which extends towards the outer circumferential surface from one end of the first extension part.

According to another aspect, a connecting point between the first extension part and the second extension part is disposed to be further recessed towards the inner space than the button part.

According to another aspect, a connecting point between the first extension part and the second extension part is disposed in the same plane as the button hole, or is disposed towards the inner space than the button hole.

According to another aspect, the operation part includes a joining cover providing an area joined to the case.

According to another aspect, the case includes a joining groove to which the operation part is joined and which includes a shape corresponding to at least a partial shape of the operation part.

According to another aspect, the operation part includes a push bar which protrudes towards the inner space from the button part.

According to another aspect, further included is a trigger which is configured to operate by the push bar, and release a stopped state of the plunger in order for the plunger to move towards one surface of the case from the stopped state adjacent to a center of the inner space.

According to another aspect, the push bar is configured to press the trigger towards the inner space, and has a density higher than that of a portion of the operation part excluding the push bar.

According to another aspect, one end of the push bar facing the inner space is formed in a curved surface.

According to another aspect, the operation part includes a support bar upwardly extending from the push bar.

According to another aspect, the operation part includes an insertion part engaged with an inner surface of the button hole, and the support bar is connected to the button part and the insertion part.

According to another aspect, the push bar forms a stepped portion from the support bar in order to protrude towards the inner space compared to the support bar.

According to another aspect, the support bar includes a bending part recessed towards the outer circumferential surface.

According to another aspect, the bending part is formed at one surface of the support bar facing the inner space.

According to another aspect, the operation part is formed of an elastic material capable of elastic deformation and restoration, and is formed of a material different from the case.

According to another aspect, a concavo-convex surface is formed on an outer surface of the button part.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 is an exploded perspective view for illustrating an applicator according to an example embodiment of the present disclosure;
FIG. 2 is an exploded perspective view for illustrating an operation part and a case according to an example embodiment of the present disclosure;
FIG. 3 is a cross-sectional view for illustrating an applicator taken along an X-Z plane according to an example embodiment of the present disclosure;
FIG. 4 is a cross-sectional view for illustrating an applicator before being triggered taken along an X-Z plane according to an example embodiment of the present disclosure;
FIG. 5 is a cross-sectional view for illustrating an applicator after being triggered taken along an X-Z plane according to an example embodiment of the present disclosure;
FIG. 6 is a cross-sectional view for illustrating a sensor unit after being attached and inserted to and in a skin of a user taken along an X-Z plane according to an example embodiment of the present disclosure;
FIG. 7 is an enlarged cross-sectional view for illustrating by enlarging a portion of an applicator according to an example embodiment of the present disclosure;
FIG. 8 is a perspective view for illustrating an inside of an operation part according to an example embodiment of the present disclosure;
FIG. 9 is a side view for illustrating a side surface of an operation part according to an example embodiment of the present disclosure;
FIG. 10 is a cross-sectional view for illustrating a portion of an applicator taken along a Y-Z plane according to an example embodiment of the present disclosure;
FIG. 11 is a cross-sectional view for illustrating an applicator before being triggered taken along a Y-Z plane according to an example embodiment of the present disclosure; and
FIG. 12 is a cross-sectional view for illustrating an applicator after being triggered taken along a Y-Z plane according to an example embodiment of the present disclosure.

### DETAILED DESCRIPTION OF EXEMPLARY EMBODIMENTS

Terms used in describing the example embodiments of the present disclosure are selected, as much as possible, from general terms that are widely used at present while taking into consideration the functions of the present disclosure, but these terms may be replaced by other terms based on intentions of those skilled in the art, judicial precedent, the advent of new technologies, or the like. Also, in a particular case, terms that are arbitrarily selected by the applicant of the present disclosure may be used. In this case, the meanings of these terms will be described in detail in corresponding description parts of the present disclosure. Accordingly, the terms used herein may be defined not based on simple names, but based on practical meanings thereof and the whole content of the present disclosure.

The suffix "portion" used for elements in this specification is provided or used interchangeably solely for ease of description, and may not have distinct meanings or functions in and of itself. In addition, in describing example embodiments included in the present disclosure, detailed descriptions of related known technologies may be omitted if it is determined that such descriptions may obscure the gist of the embodiments. Furthermore, the accompanying drawings are provided to facilitate understanding of the example embodiments included in the present disclosure and are not intended to limit the technical spirit of the present disclosure, and it should be understood that the present disclosure encompasses all modifications, equivalents, and substitutes within the scope and spirit of the invention.

Terms including ordinal numbers such as "first," "second," and the like may be used to describe various elements, but such elements are not limited by these terms, and these terms may be used merely to distinguish one element from another.

When it is stated that an element is "connected to" or "coupled to" another element, it should be understood that the element may be directly connected to or coupled to the another element, or that another element may exist therebetween. In contrast, when it is stated that an element is "directly connected to" or "directly coupled to" another element, it should be understood that no other elements exist therebetween.

In the description below, a singular expression may include a plural expression unless clearly indicated otherwise in context.

Terms such as "include" or "have" used in this specification should be understood as indicating the existence of a feature, number, step, operation, element, component, or combination thereof described in this specification, and the terms do not exclude in advance the existence or the possibility of one or more additional features, numbers, steps, operations, elements, components, or combinations thereof.

An expression "at least one of a, b, and c" described in this specification may encompass "a alone," "b alone," "c alone," "a and b," "a and c," "b and c," or "all of a, b, and c."

Hereinafter, various example embodiments of the present disclosure will be described in detail with reference to the accompanying drawings for those of ordinary skill in the art to which the present disclosure pertains to easily perform the example embodiments. However, the present disclosure may be embodied in various different forms and not limited to the example embodiments described herein.

Hereinafter, example embodiments of the present disclosure will be described in detail with reference to the drawings.

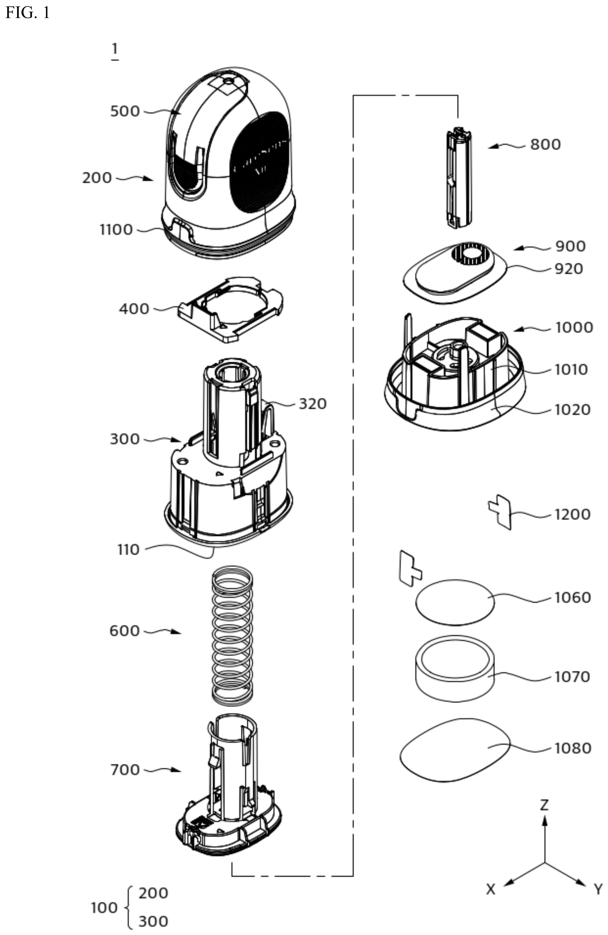

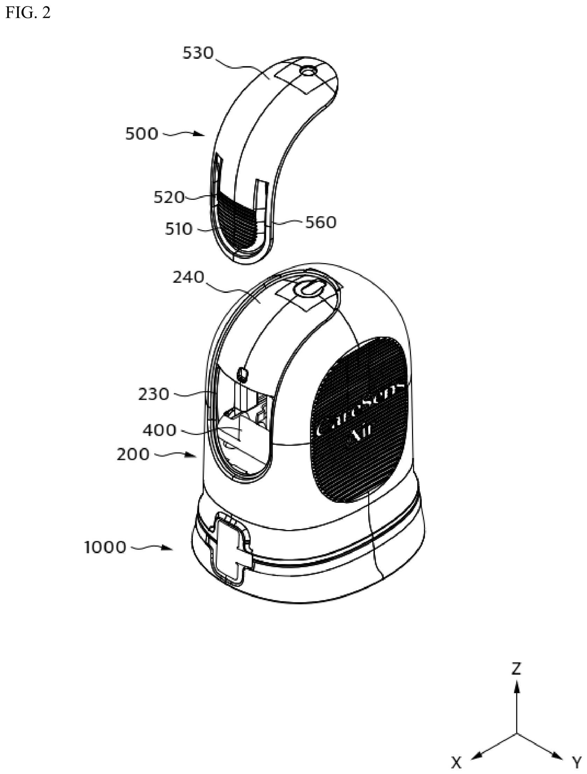

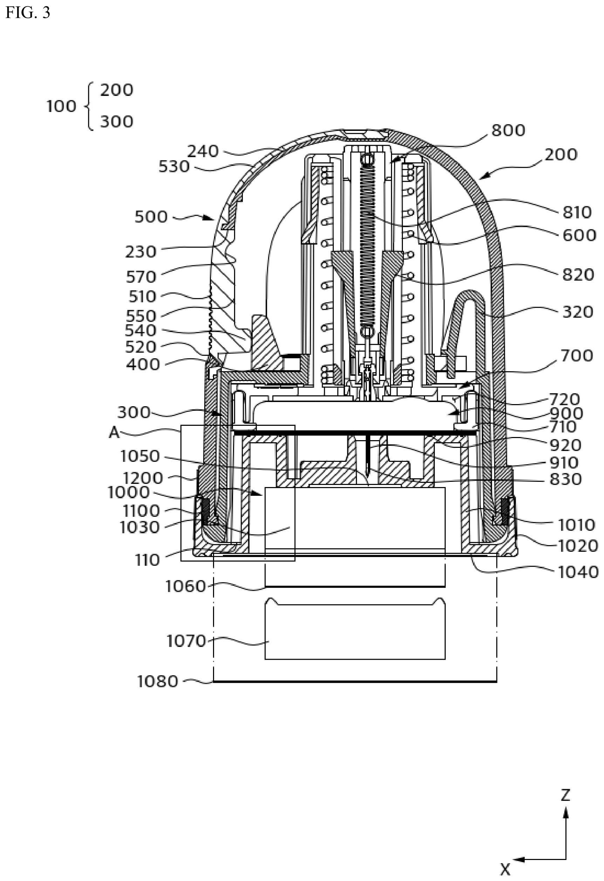

FIG. 1 is an exploded perspective view for illustrating an applicator 1 according to an example embodiment of the present disclosure, FIG. 2 is an exploded perspective view for illustrating an operation part 500 and a case 100 according to an example embodiment of the present disclosure, and FIG. 3 is a cross-sectional view for illustrating the applicator 1 taken along an X-Z plane according to an example embodiment of the present disclosure.

Referring to FIG. 1 to FIG. 3, the applicator 1 according to the present disclosure may include the case 100.

The case 100 according to an example embodiment of the present disclosure may be configured to form an outer shape while protecting configurations of the applicator 1. The case 100 may form an inner space. The case 100 may form a predetermined space which may accommodate other configurations therein. Here, the case 100 may include an opened surface. Alternatively, the case 100 may include an opening part 110. The opening part 110 may be a portion opened to one side of the case 100. The opening part 110 may be formed at an end portion in a first direction of the case 100. The opened surface of the case 100 may be opened in the first direction. The first direction may be a direction intersecting with a skin surface. The first direction may be a direction in which one side of the case 100 is opened. The first direction mentioned herein may be, for instance, a direction being in parallel with a Z axis on the drawing, and more precisely, may signify a -Z axis direction on the drawing.

The case 100 according to an example embodiment of the present disclosure may include a main case 200 and an inner case 300. The main case 200 may be a configuration forming an outer shape, and the inner case 300 may be a configuration coupled to the inside of the main case 200. The inside mentioned herein may signify an inner space formed by the main case 200. The main case 200 may be a configuration in a dome shape having an opened surface in the first direction, and the inner case 300 may be inserted and assembled in and to the opened surface. The inner case 300 may have an opened surface in the first direction. For instance, the inner case 300 may include the above-mentioned opening part 110. The opening part 110 may be opened to one side in order for a sensor unit 900 to be described hereinafter to be inserted through the opening part 110 to be assembled, or discharged in a triggering operation.

The case 100 according to an example embodiment of the present disclosure may include the operation part 500. The operation part 500 may be a portion operated by a user to operate the applicator 1. The operation part 500 may be joined to the case 100 and may seal the opened surface of the case 100. For instance, the case 100 may include a button hole 230 formed by penetrating at least a portion on an outer circumferential surface, wherein the button hole 230 may be formed by penetrating a portion of an outer circumferential surface of the main case 200. Here, the operation part 500 may seal the button hole 230.

The applicator 1 according to an example embodiment of the present disclosure may include a plunger 700. The plunger 700 may be disposed in the inner space of the case 100. Specifically, the plunger 700 may be disposed in an inner space of the inner case 300. Here, a first elastic member 600 may be inserted between the plunger 700 and the inner case 300. The first elastic member 600 may be inserted in a compressed state to expand upon triggering to move the plunger 700.

The inner case 300 according to an example embodiment of the present disclosure may guide a movement of the plunger 700 towards the opening part 110. For instance, the inner case 300 may guide a movement of the plunger 700 in the first direction. At least a portion of an inner shape of the inner case 300 may be formed to correspond to a portion of an outer shape of the plunger 700, and the inner case 300 may include a space in which the plunger 700 may move towards the opening part 110. The plunger 700 may be guided by at least a portion of an inner surface of the inner case 300 to be moved towards the opening part 110.

The plunger 700 according to an example embodiment of the present disclosure may be configured to move the sensor unit 900 towards the opening part 110. For instance, the sensor unit 900 may be moved in the first direction. The sensor unit 900 may be a configuration to obtain biometrics of the user. The plunger 700 may be moved towards the opening part 110 by the first elastic member 600, and here, may be moved towards the opening part 110 along with the sensor unit 900.

The applicator 1 according to an example embodiment of the present disclosure may further include a trigger 400. The trigger 400 may maintain a stopped state of the plunger 700, and may be configured to release the stopped state of the plunger 700 upon moving. The user may press a button part 510 of the above-described operation part 500 to move the trigger 400.

The applicator 1 according to an example embodiment of the present disclosure may include a needle assembly 800. The needle assembly 800 may move a needle 830 towards the opening part 110 upon triggering, and the moved needle 830 may puncture a portion of a skin S of the user. The needle assembly 800 may move the needle 830 in a reverse direction of a movement direction again at a specific point after triggering to retrieve the needle 830. For instance, the needle 830 may puncture a portion of the skin S of the user by moving in the first direction, before moving in a second direction again, which is a reverse direction of the first direction, to be retrieved. The second direction mentioned herein may be, for instance, a direction being in parallel with the Z axis on the drawing, and more precisely, may signify a +Z axis direction on the drawing.

In the present disclosure, although a moving direction of the plunger 700, the sensor unit 900, the needle assembly 800 and the needle 830 is described as the first direction, the same is merely an example, and the moving direction is not necessarily limited to the first direction, and may be adequately modified while being a direction in which the sensor unit 900 may be attached to the skin S of the user with the needle 830 penetrating the skin S of the user. For instance, although the opening part 110 is opened in the first direction, the needle 830 may be triggered and moved in a direction intersecting with the first direction to puncture a portion of the skin S of the user. Likewise, a direction in which a protective cap 1000 to be described hereinafter is coupled or separated to or from the case 100 and a direction in which the needle 830 is moved may intersect with each other.

The needle assembly 800 according to an example embodiment of the present disclosure may be inserted into the plunger 700. Specifically, the needle assembly 800 may be accommodated in a space disposed by upwardly protruding from the plunger 700, and the sensor unit 900 may be disposed at a lower portion of the needle assembly 800.

The applicator 1 according to an example embodiment of the present disclosure may include the protective cap 1000. The protective cap 1000 may shield the opening part 110 of the case 100. The protective cap 1000 may be formed to surround at least a portion of an outer circumferential surface of the opening part 110 of the case 100. For instance, the protective cap 1000 may be formed to surround at least a portion of an outer circumferential surface of an end portion disposed in the first direction of an end portion of the case 100. The protective cap 1000 may include a stopper shape in a concave shape in order for the opening part 110 of the case 100 to be inserted therein. At least a portion of the opening part 110 of the case 100 may be inserted into the protective cap 1000.

The protective cap 1000 according to an example embodiment of the present disclosure may include a column part 1010 and an outer wall part 1020. The column part 1010 may be a portion inserted into the case 100, and the outer wall part 1020 may be a portion surrounding the outer circumferential surface of the case 100. The column part 1010 may surround the inside of the opening part 110, and the outer wall part 1020 may surround the outside of the opening part 110. For instance, between the column part 1010 and the outer wall part 1020 of the protective cap 1000, a groove may be formed in order for an end portion in the first direction of the case 100 to be inserted therein. The column part 1010 may surround the inside of the end portion in the first direction of the case 100, and the outer wall part 1020 may shield the opened surface of the case 100 by surrounding the outside of the end portion in the first direction of the case 100.

The applicator 1 according to an example embodiment of the present disclosure may include a sealing member 1100. The sealing member 1100 may be disposed between the case 100 and the protective cap 1000 to seal the inner space of the case 100. For instance, the sealing member 1100 may fill an empty space which may be formed between the protective cap 1000 and the case 100. Specifically, the outer wall part 1020 of the protective cap 1000 may come in contact with the sealing member 1100 on the inside. The sealing member 1100 may be disposed between the outer circumferential surface of the opening part 110 of the case 100 and the protective cap 1000. The sealing member 1100 may fill an empty space between the main case 200 and the outer wall part 1020 at a portion adjacent to the opening part 110.

The sealing member 1100 according to an example embodiment of the present disclosure may include an elastic material. The sealing member 1100 may be compressively deformed by the protective cap 1000 to fill a gap between the case 100 and the protective cap 1000. For instance, a material such as rubber may be applied as the elastic material.

The sealing member 1100 according to an example embodiment of the present disclosure may include a ring shape which is continuously connected. The ring shape of the sealing member 1100 may be disposed to surround at least a portion of the outer circumferential surface of the case 100.

The protective cap 1000 according to an example embodiment of the present disclosure may be separated and coupled in a direction facing the case 100 and the opening part 110 and in a reverse direction thereof. For instance, the protective cap 1000 may be separated and coupled in the first direction and the second direction. The sealing member 1100 may be disposed on a plane intersecting with a direction facing the opening part 110. The sealing member 1100 may be spaced apart from the opening part 110 by a uniform interval. The sealing member 1100 may provide a uniform sealing force between the protective cap 1000 and the case 100.

The above-described operation part 500 according to an example embodiment of the present disclosure may include the elastic material to be able to be operated by the user, and the operation part 500 may be joined to the case 100 to seal the button hole 230 of the case 100. Further, the sealing member 1100 may be joined to the case 100 to seal a gap between the protective cap 1000 and the case 100. The operation part 500 and the sealing member 1100 may be formed of an identical material, and may be formed of another material than the case 100. Here, the operation part 500 and the sealing member 1100 may be formed along with the case 100. For instance, by a double injection method, the operation part 500 and the sealing member 1100 may be manufactured along with the case 100. Nevertheless, because such a manufacturing method is merely an example, a manufacturing method of applying an adhesive onto the case 100 to attach the operation part 500 to the sealing member 1100 is also possible.

The protective cap 1000 according to an example embodiment of the present disclosure may include a third vent 1050. The third vent 1050 may be a hole communicating with the inside of the case 100. In a state in which the protective cap 1000 is coupled to the case 100, the inner space of the case 100 may be in a state of being sealed at a remaining portion excluding the third vent 1050. Here, sterilization gas may be injected through the third vent 1050. The injected sterilization gas may sterilize the inner space of the case 100.

The protective cap 1000 according to an example embodiment of the present disclosure may include a first cover member 1060. The first cover member 1060 may seal the third vent 1050. The first cover member 1060 may perform sealing by covering an outer surface of the third vent 1050.

The protective cap 1000 according to an example embodiment of the present disclosure may include a second cover member 1080. The second cover member 1080 may seal the first cover member 1060. The second cover member 1080 may perform sealing by covering the outside of the first cover member 1060.

The protective cap 1000 according to an example embodiment of the present disclosure may include a dehumidification member 1070. The dehumidification member 1070 may be a configuration to remove humidity. The dehumidification member 1070 may be disposed between the first cover member 1060 and the second cover member 1080.

The third vent 1050, the first cover member 1060, the dehumidification member 1070, and the second cover member 1080 according to an example embodiment of the present disclosure may be sequentially disposed in one direction. For instance, the third vent 1050, the first cover member 1060, the dehumidification member 1070, and the second cover member 1080 may be sequentially disposed in the first direction.

The first cover member 1060 and the second cover member 1080 according to an example embodiment of the present disclosure may be formed of materials different from each other.

The first cover member 1060 according to an example embodiment of the present disclosure may be formed of a material having air permeability and a bacterial blocking property for blocking passage of bacteria. The first cover member 1060 may include a porous filter structure. Therefore, even when sterilization gas is injected in a state in which the first cover member 1060 has sealed the third vent 1050, the sterilization gas may pass through the first cover member 1060 to be injected into the inner space of the case 100 through the third vent 1050. Nevertheless, the porous filter structure of the first cover member 1060 may be formed to be sufficiently small in order for bacteria not to pass. Therefore, external bacteria may not pass through the first cover member 1060.

The second cover member 1080 according to an example embodiment of the present disclosure may be formed of a dampproof material. For instance, the second cover member 1080 may be an aluminum film. Therefore, external humidity may not pass through the second cover member 1080.

The first cover member 1060, the dehumidification member 1070, and the second cover member 1080 according to an example embodiment of the present disclosure may be accommodated in the protective cap 1000 in a triple structure, and may cause an inner environment of the case 100 to be maintained in a sterilized, dehumidified, and sealed state. In a state in which the third vent 1050 is covered with the first cover member 1060, sterilization gas may be injected in order to cause the inner space of the case 100 to be in a sterilized state before the dehumidification member 1070 is disposed, and the second cover member 1080 covers the outside thereof in order to form a structure for preventing external bacteria or humidity from infiltrating into the inner space of the case 100.

The protective cap 1000 according to an example embodiment of the present disclosure may include a first accommodating part 1030. The first accommodating part 1030 may be a portion of the protective cap 1000 which is inwardly recessed to accommodate the dehumidification member 1070. Specifically, the first accommodating part 1030 may be a space which is formed by being inwardly recessed from an end portion in the first direction of the column part 1010. The third vent 1050 may be formed by penetrating an upper surface of the first accommodating part 1030. The first cover member 1060 may cover an upper surface of the first accommodating part 1030. Beneath the same, in a remaining space of the first accommodating part 1030, the dehumidification member 1070 may be disposed.

The protective cap 1000 according to an example embodiment of the present disclosure may include a second accommodating part 1040. The second accommodating part 1040 may be a portion of the protective cap 1000 in which the second cover member 1080 is accommodated. The second accommodating part 1040 may be formed to be wider and thinner than the first accommodating part 1030. The second cover member 1080 may be formed to be wider than the first cover member 1060 and the dehumidification member 1070. The second accommodating part 1040 and the first accommodating part 1030 may form a stair structure with each other. Referring to FIG. 3, the second accommodating part 1040 may be disposed at a lower portion of the first accommodating part 1030, and may be formed to be wider than the first accommodating part 1030 in a direction perpendicular to the first direction. For instance, a direction perpendicular to the first direction may be a third direction. The third direction may be a direction being in parallel with an X axis direction or a Y axis direction on the drawing.

As the first cover member 1060, the dehumidification member 1070, and the second cover member 1080 are accommodated in the protective cap 1000 according to an example embodiment of the present disclosure, a packaging for sterilization, dehumidification, and sealing of the applicator 1 may be simplified with the single protective cap 1000, and a size of the packaging may also be reduced.

The applicator 1 according to an example embodiment of the present disclosure may further include a display member 1200.

Before the applicator 1 according to an example embodiment of the present disclosure is used, an inner space of the applicator 1 should be maintained in a sterilized, dehumidified, and sealed state. In case that the protective cap 1000 of the applicator 1 is removed from the case 100, a state of maintaining sterilization, dehumidification, and sealing of the inner space of the case 100 may be broken, and re-use of the applicator 1 may be impossible.

The display member 1200 according to an example embodiment of the present disclosure is a configuration to identify that the state of maintaining sterilization, dehumidification, and sealing thereof is broken, and may be disposed in order for, when the protective cap 1000 is removed once, a trace thereof to remain.

The display member 1200 according to an example embodiment of the present disclosure may be successively attached to the protective cap 1000 and the case 100. The single display member 1200 may be attached by being connected to a portion of an outer surface of the protective cap 1000 and a portion of an outer surface of the case 100. The display member 1200 may be attached to a connecting point between the protective cap 1000 and the case 100.

The display member 1200 according to an example embodiment of the present disclosure may be disposed in order for at least one portion thereof to be broken or in order to leave a trace at the protective cap 1000 or the case 100 in a case in which the protective cap 1000 is removed.

The display member 1200 according to an example embodiment of the present disclosure may include a breaking line of which a portion is broken for easy breaking. In case that the protective cap 1000 is removed, the display member 1200 may be broken along the breaking line by external force. A portion of the broken display member 1200 may be attached to the protective cap 1000, and a remaining portion may be attached to the case 100.

The display member 1200 according to another example embodiment of the present disclosure may, when being removed from a target to which the same is attached, be disposed to leave a trace such as an ink stain, an adhesive or the like at the target. For instance, when the protective cap 1000 is removed, the display member 1200 is attached to the protective cap 1000, and, at a portion of the case 100 to which the display member 1200 had been attached, a trace such as an ink stain and the like may remain.

The above-mentioned descriptions with respect to the display member 1200 are merely some examples of various means by which the display member 1200 leaves a trace, and may be appropriately changed within a range for achieving an objective of the present disclosure.

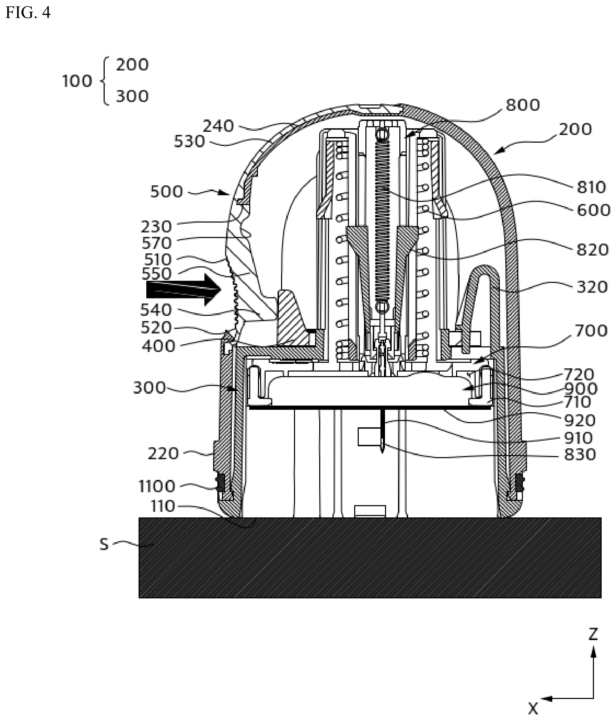

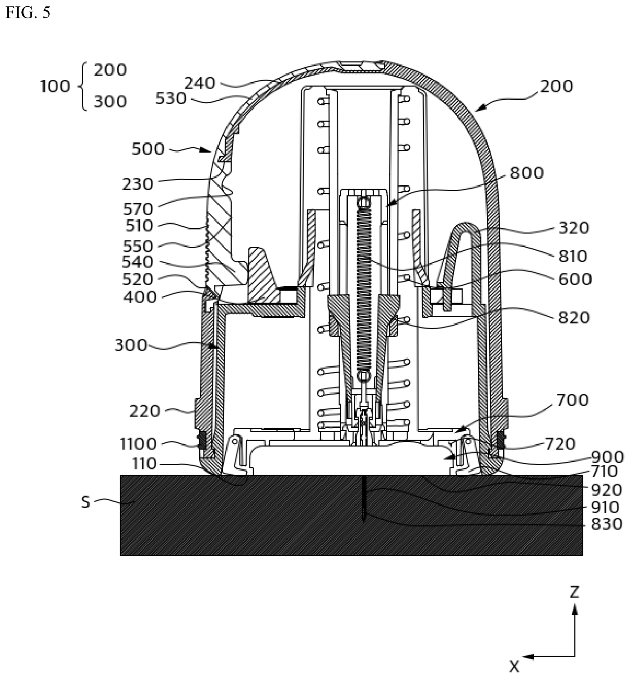

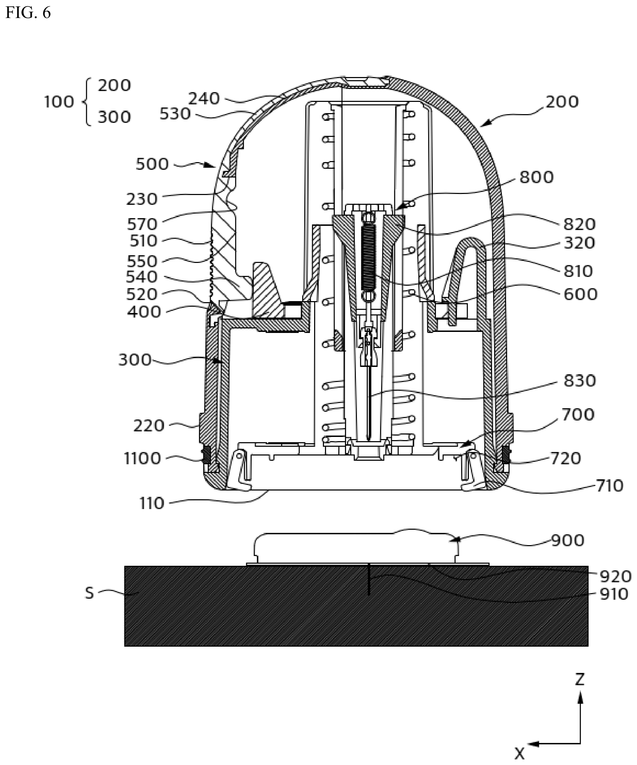

FIG. 4 is a cross-sectional view for illustrating the applicator 1 before being triggered taken along an X-Z plane according to an example embodiment of the present disclosure, FIG. 5 is a cross-sectional view for illustrating the applicator 1 after being triggered taken along an X-Z plane according to an example embodiment of the present disclosure, and FIG. 6 is a cross-sectional view for illustrating the sensor unit 900 after being attached and inserted to and in the skin S of a user taken along an X-Z plane according to an example embodiment of the present disclosure.

Referring to FIG. 4 to FIG. 6, a triggering mechanism of the applicator 1 is described.

FIG. 4 illustrates the applicator 1 before being triggered, and, referring to FIG. 4, in order to use the applicator 1, a user may remove the protective cap 1000, and may locate the applicator 1 in order for the opening part 110 of the case 100 to come in contact with the skin S of the user. For instance, one end in the first direction of the case 100 may come in contact with the skin S of the user.

The plunger 700 according to an example embodiment of the present disclosure may be gripping the sensor unit 900. A socket 720 exists at a lower portion of the plunger 700 in a direction facing the opening part 110, and the socket 720 may form a predetermined space to accommodate the sensor unit 900. Here, around the socket 720, at least one hook 710 may be formed. One end of the hook 710 may be bent, and the bent portion of the hook 710 may support at least a portion of the sensor unit 900. The sensor unit 900 supported by the hook 710 may maintain a state of being fixed in the socket 720.

The plunger 700 according to an example embodiment of the present disclosure may be in a state of being stopped at a central height or a center of the case 100. Here, the first elastic member 600 disposed between the plunger 700 and the inner case 300 may be in a compressed state. The trigger 400 is in a state of being disposed on a relatively left side on the drawing before triggering, and is engaged with at least a portion of the plunger 700, and may fix the plunger 700 in order for the plunger 700 not to be moved by the first elastic member 600. In other words, the trigger 400 may maintain the plunger 700 in a stopped state.

The sensor unit 900 according to an example embodiment of the present disclosure may include a probe 910 inserted into a human body, and the probe 910 may protrude towards one side. For instance, the probe 910 may protrude in a direction facing the opening part 110 in a state before being triggered. An adhesive layer 920 is formed at a lower portion of a body of the sensor unit 900, and the adhesive layer 920 may be formed to be wider than the sensor unit 900. The adhesive layer 920 may be attached to a human body, and the sensor unit 900 may be fixed to the skin S.

The needle assembly 800 according to an example embodiment of the present disclosure may be accommodated in an upper part of the plunger 700. The needle 830 may have one end which is pointed to puncture the skin S, and may accommodate the probe 910 by forming a hollow space therein.

The user may locate the applicator 1 at a using location before pressing the button part 510 of the operation part 500 to trigger the applicator 1.

FIG. 5 illustrates a state after being triggered, and, referring to FIG. 5, the plunger 700 may be moved towards the opening part 110 to attach the sensor unit 900 to the skin S.

A user may press the button part 510 towards the inner space of the case 100. At least a partial area of the button part 510 may be disposed in the inner space through the button hole 230. Here, a push bar 540 disposed on the inside of the button part 510 may be moved towards the inner space. One end of the push bar 540 may pressurize the trigger 400, and the trigger 400 may be moved towards the inner space. One surface of the trigger 400 may come in contact with the push bar 540, and the opposite one surface thereof may come in contact with a return member 320. The return member 320 is a configuration disposed at the inner case 300 and may be elastically deformed to move the trigger 400 to an original location. For instance, in case that the user releases the trigger 400 at the button part 510, the same may return to the original location again by the return member 320.

The trigger 400 which is moved towards the inner space may release the stopped state of the plunger 700. By expansion of the compressed first elastic member 600, the plunger 700 may be moved towards the opening part 110. For instance, the plunger 700 may be moved in the first direction.

The sensor unit 900 and the needle assembly 800 according to an example embodiment of the present disclosure may be moved towards the opening part 110 along with the plunger 700.

The needle 830 of the needle assembly 800 according to an example embodiment of the present disclosure may be moved towards the opening part 110 to puncture the skin S by a predetermined depth. The probe 910 disposed in the needle 830 may be inserted into a hole of the skin S formed by the needle 830. The adhesive layer 920 of the sensor unit 900 may be attached to the surface of the skin S, and the sensor unit 900 may be fixed at the skin S in a state of inserting the probe 910 into the skin S.

The inner case 300 according to an example embodiment of the present disclosure may include a guide wall at an inner wall, and the guide wall may guide a movement of the hook 710 along a moving path of the hook 710. The guide wall may outwardly widen towards the opening part 110. For instance, the guide wall may outwardly widen in the first direction. One surface of the hook 710 may be moved in the first direction while coming in contact with the guide wall. At an end portion in the first direction, the hook 710 may be rotated. By a rotation of the hook 710, the bent portion of the hook 710 supporting a lower portion of the sensor unit 900 may move away from the sensor unit 900, and the hook 710 may release fixation of the sensor unit 900.

FIG. 6 illustrates a state in which the sensor unit 900 is attached to the skin S. Referring to FIG. 5 and FIG. 6, the needle 830 may be retrieved after attaching the sensor unit 900 to the skin S.

The needle assembly 800 according to an example embodiment of the present disclosure may include a retrieving part 820. One end of the retrieving part 820 may fix the needle 830. The retrieving part 820 may be fixed by being latched at a portion of a frame of the needle assembly 800. At a point in time in which the needle assembly 800 is moved towards the opening part 110 in order for the needle 830 to puncture the skin S by a predetermined depth, the retrieving part 820 may be pressurized in an inward direction by the inner case 300. Here, the retrieving part 820 may be released after having been latched at the frame of the needle assembly 800.

The needle assembly 800 according to an example embodiment of the present disclosure may include a second elastic member 810. The second elastic member 810 may be coupled to the retrieving part 820 in a stretched state. In case that the retrieving part 820 is released after having been latched at the frame of the needle assembly 800, the second elastic member 810 may pull the retrieving part 820 in a reverse direction (for instance, the second direction) to a moving direction.

By the retrieving part 820 according to an example embodiment of the present disclosure, the needle 830 may be retrieved in a reverse direction (for instance, the second direction) of a moving direction to be accommodated in the needle assembly 800. The retrieved needle 830 may not be exposed to the outside in order to prevent a user from being injured. As described above, description of a moving direction of the needle assembly 800 and the needle 830 being the first direction is merely an example, and the moving direction of the needle assembly 800 and the needle 830 is possible to be a direction intersecting with the first direction.

Mechanisms and configurations related to triggering of the applicator 1 described in FIG. 4 to FIG. 6 may be merely an example. Accordingly, even when a triggering mechanism other than the same is applied to the applicator 1, a configuration related to maintaining of sterilization, dehumidification, and sealing of the present disclosure may be identically applied.

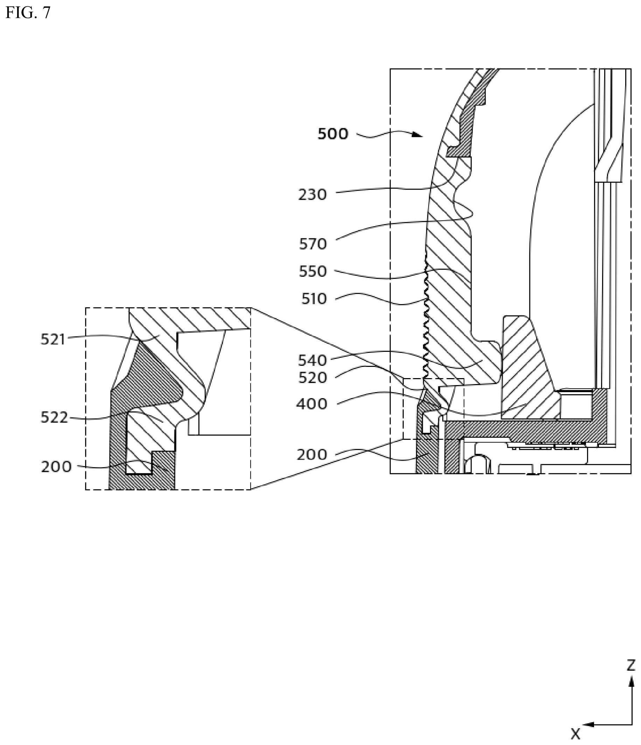

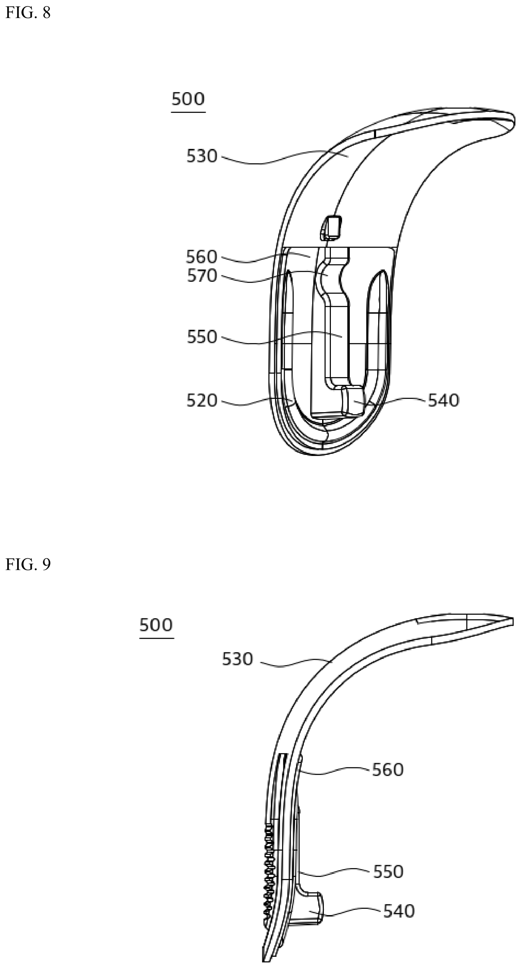

FIG. 7 is an enlarged cross-sectional view for illustrating by enlarging a portion of the applicator 1 according to an example embodiment of the present disclosure, FIG. 8 is a perspective view for illustrating an inside of the operation part 500 according to an example embodiment of the present disclosure, and FIG. 9 is a side view for illustrating a side surface of the operation part 500 according to an example embodiment of the present disclosure.

By referring to FIG. 1 to FIG. 6, along with FIG. 7 to FIG. 9, a configuration of the operation part 500 is described in detail.

The operation part 500 according to an example embodiment of the present disclosure may include the button part 510. The button part 510 may be moved towards the inner space through the button hole 230. The inner space mentioned herein may signify the inner space of the case 100. The button part 510 may be easily and elastically deformed to be easily moved towards the inner space through the button hole 230 when pressurized. Further, the button part 510 may be more easily moved by an edge groove part 520 to be described hereinafter.

The button part 510 according to an example embodiment of the present disclosure may form a concavo-convex surface on an outer surface of the button part 510. The user may easily find the button part 510 by looking at the concavo-convex surface. Further, while the user pressurizes the button part 510, a slip may be prevented by the concavo-convex surface.

The operation part 500 according to an example embodiment of the present disclosure may include a joining cover 530. The joining cover 530 may provide an area joined to the case 100. The joining cover 530 is formed by extending around the button part 510, and one surface thereof may be accommodated in the case 100 or joined to the case 100.

The case 100 according to an example embodiment of the present disclosure may include a joining groove 240, and the joining groove 240 may be a portion to which the operation part 500 is joined. The joining groove 240 may include a shape corresponding to a shape of at least a portion of the operation part 500. For instance, the joining groove 240 may accommodate the joining cover 530. The joining cover 530 may include a shape corresponding to a shape of the joining groove 240.

The operation part 500 according to an example embodiment of the present disclosure may include the edge groove part 520. The edge groove part 520 may be disposed in order for the button part 510 to be easily moved.

The edge groove part 520 according to an example embodiment of the present disclosure may be formed by being recessed. The edge groove part 520 may be formed by being recessed towards the inner space from the outer circumferential surface of the case 100. The edge groove part 520 may include a groove of which at least a portion is dented towards the inner space, or may include a shape of being folded into the inner space.

The edge groove part 520 according to an example embodiment of the present disclosure may be formed to be thinner than a periphery thereof. For instance, the edge groove part 520 may have a thickness thinner than a thickness of the operation part 500.

The edge groove part 520 may be formed to be thinner than a portion of the other operation part 500 connected to the edge groove part 520 to be easily and elastically deformed.

At least a portion of the edge groove part 520 according to an example embodiment of the present disclosure may be formed along a periphery of the button part 510. For instance, in case that the button part 510 is formed in a circular shape, the edge groove part 520 may be formed along a portion of a circular shape of a periphery of the button part 510. Specifically, the edge groove part 520 is formed at a periphery of a lower portion and a side portion of the button part 510, and may include a U-letter shape which rectilinearly extends from the side portion of the button part 510 to an upper portion thereof. Nevertheless, a shape of the edge groove part 520 is not limited thereto, and may be deformed in various shapes such as being formed in an O-letter shape along an edge of the button part 510.

The edge groove part 520 according to an example embodiment of the present disclosure may include a first extension part 521 and a second extension part 522. The first extension part 521 may be a portion to extend towards the inner space from the button part 510. The first extension part 521 may be connected towards the inner space from a periphery of the button part 510. The second extension part 522 may extend towards an outer circumferential surface from one end of the first extension part 521. The first extension part 521 may extend towards the inner space before being bent towards the outer circumferential surface which is an opposite direction thereto to be connected to the second extension part 522. The first extension part 521 and the second extension part 522 may form a V or U shape.

The first extension part 521 and the second extension part 522 according to an example embodiment of the present disclosure may be respectively formed in plural. The drawings illustrate a shape in which the first extension part 521 and the second extension part 522 are formed by one respectively, but this is merely an example. Therefore, the first extension part 521 and the second extension part 522 may be formed by two respectively to form a W shape.

A connecting point between the first extension part 521 and the second extension part 522 according to an example embodiment of the present disclosure may be disposed in the same plane as the button hole 230, or may be disposed to be further recessed towards the inner space than the button part 510. The connecting point between the first extension part 521 and the second extension part 522 may be a point at which the first extension part 521 and the second extension part 522 meet at one end towards the inner space. Alternatively, the connecting point may be disposed towards the inner space compared to the button hole 230. A connecting part may be formed by being sufficiently inserted into the inner space in order for the button part 510 to be easily inserted into the inner space.

The operation part 500 according to an example embodiment of the present disclosure may include the push bar 540. The push bar 540 may protrude towards the inner space from the button part 510. The push bar 540 may protrude towards the inner space to come in contact with the trigger 400. The trigger 400 may be operated by the push bar 540, and may release a stopped state of a plunger in order for the plunger to be moved towards one surface of the case 100 from the stopped state adjacent to a center of the inner space.

In case that the button part 510 according to an example embodiment of the present disclosure is pressurized and is moved towards the inner space, the push bar 540 may be moved towards the inner space along therewith. The push bar 540 may push the trigger 400 towards the inner space to release the stopped state of the plunger. In other words, the push bar 540 may be configured to press the trigger 400 towards the inner space.

The push bar 540 according to an example embodiment of the present disclosure may be formed to stably push the trigger 400. For instance, the push bar 540 may be formed to have a density higher than those of other portions of the operation part 500. As described above, the operation part 500 may be formed of an elastic material which may be elastically deformed and restored, and may be formed of a material different from that of the case 100. In case that the operation part 500 is formed in a double injection method along with the case 100, sufficient time and pressure may be applied to the push bar 540 in order to form a high density at the push bar 540. The push bar 540 may be formed to have a high density, and may have a sufficient stiffness to apply external force to the trigger 400. The push bar 540 may be formed to have a density higher than that of a portion of the operation part 500 excluding the push bar 540.

Further, one end of the push bar 540 facing the inner space according to an example embodiment of the present disclosure may be formed in a curved surface. The push bar 540 is formed of an elastic material, and may be elastically deformed. One end of the push bar 540 is formed in a curved surface in order to gradually widen a contact area with the trigger 400 while applying external force to the trigger 400.

The operation part 500 according to an example embodiment of the present disclosure may include a support bar 550. The operation part 500 is formed of an elastic material, and the button part 510 may be formed to be thin. Accordingly, while a button is pressurized, the button part 510 may be distorted in order for the push bar 540 to fail at aiming at the trigger 400 and miss the same. The support bar 550 may be disposed in order to stably hold a position of the push bar 540. The support bar 550 may upwardly extend from the push bar 540. The support bar 550 includes a stick shape to sufficiently support the push bar 540, and may be connected to the inside of an operation cover and the button part 510.

The support bar 550 according to an example embodiment of the present disclosure may include a bending part 570. The bending part 570 may be disposed in order for the button part 510 to be easily moved towards the inner space, or may be disposed in order for the push bar 540 to be easily moved towards the inner space.

The bending part 570 according to an example embodiment of the present disclosure may be recessed towards an outer circumferential surface. For instance, the bending part 570 may be recessed towards an outer circumferential surface from one surface of the support bar 550 facing the inner space. In a case of pressurizing the button part 510, the support bar 550 may be easily bent around the bending part 570. Therefore, the button part 510 or the push bar 540 may be easily moved towards the inner space by the bending of the support bar 550.

The push bar 540 according to an example embodiment of the present disclosure may protrude towards the inner space compared to the support bar 550. The push bar 540 may form a stepped portion with the support bar 550.

The operation part 500 according to an example embodiment of the present disclosure may include an insertion part 560. The operation part 500 may be engaged with an inner surface of the button hole 230. The operation part 500 may be formed on the inside of the button hole 230. The operation part 500 may include a shape corresponding to the button hole 230. Here, the support bar 550 may be connected to the button part 510 and the insertion part 560. The operation part 500 may be formed to be thicker than the button part 510.

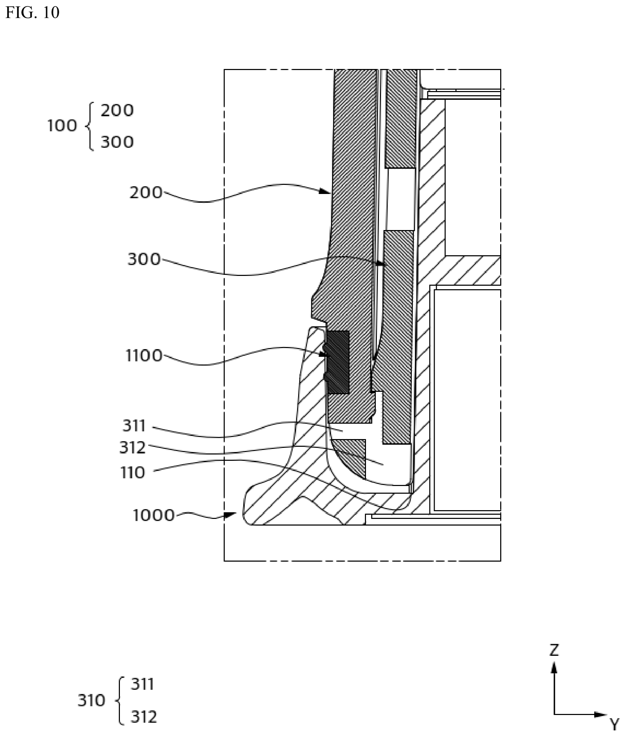

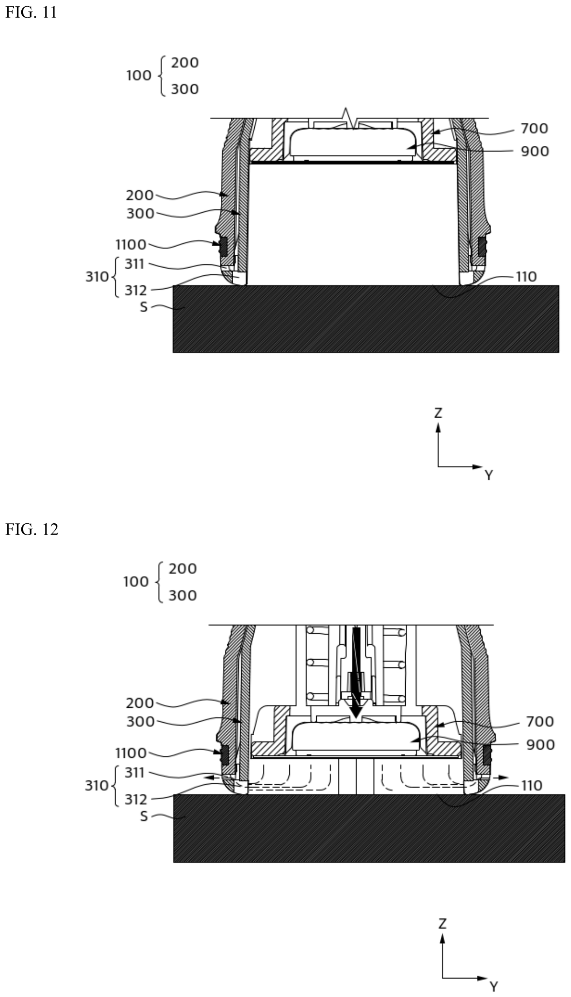

FIG. 10 is a cross-sectional view for illustrating a portion of the applicator 1 taken along a Y-Z plane according to an example embodiment of the present disclosure, FIG. 11 is a cross-sectional view for illustrating the applicator 1 before being triggered taken along a Y-Z plane according to an example embodiment of the present disclosure, and FIG. 12 is a cross-sectional view for illustrating the applicator 1 after being triggered taken along a Y-Z plane according to an example embodiment of the present disclosure.

Referring to FIG. 10 to FIG. 12, a vent 310 according to an example embodiment of the present disclosure may provide a path through which the air in the inner space of the case 100 may be discharged to the outside. Specifically, the vent 310 may provide a path in order for the air pushed by the plunger 700 moving towards the opening part 110 in a triggered state to be discharged to the outside of the case 100.

The vent 310 according to an example embodiment of the present disclosure may be spaced apart from the sealing member 1100 towards the opening part 110. For instance, the vent 310 may be spaced apart in the first direction from the sealing member 1100. The vent 310 is a hole disposed in order for the air in the inner space of the case 100 to be discharged to the outside and may communicate with the outside. It is possible that, before the applicator 1 is used, the inner space of the case 100 should be maintained in a sealed state, and that the vent 310 should be sealed. Accordingly, in a state in which the protective cap 1000 is coupled to the case 100, in order for the vent 310 not to communicate with the outside, the vent 310 may be disposed in the first direction compared to the sealing member 1100. The sealing member 1100 seals the gap between the protective cap 1000 and the case 100, and the vent 310 disposed in the first direction compared to the sealing member 1100 may be sealed.

A first vent 311 and a second vent 312 according to an example embodiment of the present disclosure may form a stair structure with each other. The first vent 311 and the second vent 312 may form a stepped protrusion. The first vent 311 and the second vent 312 may form a stepped protrusion towards the opening part 110. For instance, the first vent 311 and the second vent 312 may form a stepped protrusion in the first direction. The second vent 312 may be disposed to be closer to the opening part 110 than the first vent 311, and may be disposed on the inside. While being used, at least one surface of the second vent 312 may come in contact with the skin S of a user, and may be disposed at an end portion of the first direction of the case 100. At least a portion of the second vent 312 may include a downwardly opened shape. Accordingly, the air pushed by the plunger 700 moving in the first direction may be discharged to the outside through the vent 310 up to the last of the triggered state.

At least a portion of the vent 310 according to an example embodiment of the present disclosure may be formed between the sealing member 1100 and the opening part 110 of the case 100. For instance, the first vent 311 may be disposed between an end portion in the first direction and the sealing member 1100. The first vent 311 may be formed by being spaced apart from an end portion in the first direction of the case 100, and may prevent a stiffness of the end portion in the first direction of the case 100 from weakening.

The first vent 311 according to an example embodiment of the present disclosure may be a hole facing the outside of the case 100. For instance, when the main case 200 and the inner case 300 are coupled, the first vent 311 may include a shape which is radially opened based on a longitudinal direction of the case 100. For instance, the first vent 311 may be opened in a direction being in parallel with the third direction on the drawing. Here, the second vent 312 may also be opened in a direction being in parallel with the third direction.

The first vent 311 and the second vent 312 according to an example embodiment of the present disclosure may be connected to each other. For instance, the first vent 311 and the second vent 312 may be connected to each other while forming a layer. At least a portion of the first vent 311 may be disposed on at least a portion of the second vent 312 in order for the same to mutually communicate.

The vent 310 according to an example embodiment of the present disclosure may be formed in plural. For instance, the vent 310 may be formed by at least one pair. The at least one pair of vents 310 may be disposed to face each other. The air of the inner space of the case 100 may be discharged through the at least one pair of vents 310 in a balanced manner.

The above-described shape, number and location of the vents 310 are merely an example. Therefore, it is possible that the first vent 311 and the second vent 312 are formed by being rectilinearly penetrated or diagonally penetrated, and it is also possible that, because the vents 310 do not necessarily have to form a pair, the same are formed in an odd number.

According to example embodiments, it is possible to maintain an inner space of an applicator in a sterilized, dehumidified, and sealed state.

Furthermore, according to example embodiments, it is possible to simplify a packaging which may maintain an inner space of an applicator in a sterilized, dehumidified, and sealed state, and reduce a size thereof.

Furthermore, according to example embodiments, it is possible to reduce costs in transportation of an applicator and increase efficiency by a reduced size of a packaging of an applicator.

While the present disclosure is illustrated and described with reference to desired example embodiments to exemplify the principle of the present disclosure, the present disclosure is not limited to the configuration and operation exactly as shown and described. Rather, it will be understood by those skilled in the art that various modifications and alterations may be made to the present disclosure without departing from the spirit and scope of the appended claims.

## Claims

1. An applicator (1), comprising:
a case (100) which forms an inner space, comprises an opening part (110) opened to one side, and comprises a button hole (230) penetrating at least a portion of an outer circumferential surface;
an operation part (500) which seals the button hole (230); and
a plunger (700) which is disposed in the inner space of the case (100), and is configured to move a sensor unit (900) towards the opening part (110) by an operation of the operation part (500),
wherein the operation part (500) comprises:
a button part (510) of which at least a partial area is disposed in the inner space through the button hole (230); and
an edge groove part (520) which is configured to facilitate movement of the button part (510).

2. The applicator (1) of claim 1, wherein the edge groove part (520) is formed to be recessed towards the inner space from the outer circumferential surface.

3. The applicator (1) of claim 1 or 2, wherein at least a portion of the edge groove part (520) is formed along a periphery of the button part (510), and
wherein edge groove part (520) comprises a first extension part (521) which extends towards the inner space from the button part (510) and a second extension part (522) which extends towards the outer circumferential surface from one end of the first extension part (521).

4. The applicator (1) of claim 3, wherein a connecting point between the first extension part (521) and the second extension part (522) is disposed to be further recessed towards the inner space than the button part (510).

5. The applicator (1) of claim 3, wherein a connecting point between the first extension part (521) and the second extension part (522) is disposed in the same plane as the button hole (230), or is disposed towards the inner space than the button hole (230).

6. The applicator (1) of any one of the preceding claims, wherein the operation part (500) comprises a joining cover (530) providing an area joined to the case (100), and
wherein the case (100) comprises a joining groove (240) to which the operation part (500) is joined and which comprises a shape corresponding to at least a partial shape of the operation part (500).

7. The applicator (1) of any one of the preceding claims, further comprising a trigger (400) which is configured to operate by the push bar (540), and release a stopped state of the plunger (700) in order for the plunger (700) to move towards one surface of the case (100) from the stopped state adjacent to a center of the inner space, and
wherein the operation part (500) comprises a push bar (540) which protrudes towards the inner space from the button part (510).

8. The applicator (1) of claim 7, wherein the push bar (540) is configured to press the trigger (400) towards the inner space, and has a density higher than that of a portion of the operation part (500) excluding the push bar (540).

9. The applicator (1) of claim 7 or 8, wherein one end of the push bar (540) facing the inner space is formed in a curved surface.

10. The applicator (1) of any one of claims 7 to 9, wherein the operation part (500) comprises a support bar (550) upwardly extending from the push bar (540).

11. The applicator (1) of claim 10, wherein the operation part (500) comprises an insertion part (560) engaged with an inner surface of the button hole (230), and
wherein the support bar (550) is connected to the button part (510) and the insertion part (560).

12. The applicator (1) of claim 10 or 11, wherein the push bar (540) forms a stepped portion from the support bar (550) in order to protrude towards the inner space compared to the support bar (550).

13. The applicator (1) of any one of claims 10 to 12, wherein the support bar (550) comprises a bending part (570) recessed towards the outer circumferential surface, and
wherein the bending part (570) is formed at one surface of the support bar (550) facing the inner space.

14. The applicator (1) of any one of the preceding claims, wherein the operation part (500) is formed of an elastic material capable of elastic deformation and restoration, and is formed of a material different from the case (100).

15. The applicator (1) of any one of the preceding claims, wherein a concavo-convex surface is formed on an outer surface of the button part (510).
